(19) Europäisches Patentamt / European Patent Office / Office européen des brevets

(11) **EP 3 550 027 A1**

(12) **EUROPEAN PATENT APPLICATION**

(43) Date of publication:
09.10.2019 Bulletin 2019/41

(51) Int Cl.:
*C12P 19/04* (2006.01)

(21) Application number: 18165787.5

(22) Date of filing: 04.04.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Clariant International Ltd**
**4132 Muttenz (CH)**

(72) Inventors:
• **SIEKER, Tim**
**82152 Krailling (DE)**

• **DANZER, Lukas**
**85368 Eching (DE)**
• **DENNEWALD, Danielle**
**80798 München (DE)**
• **ZAVREL, Michael**
**82140 Olching (DE)**
• **DIETZ, Heiko**
**27638 Wurster Nordseeküste (DE)**

(74) Representative: **Graser, Konstanze**
**Clariant Produkte (Deutschland) GmbH**
**IPM / Patent & License Management**
**Arabellastrasse 4a**
**81925 München (DE)**

(54) **PROCESS FOR THE FERMENTATION OF ASCOMYCOTA**

(57)     The present invention refers to a process for the fermentation of *Ascomycota,* a beta-glucan produced by the process and the use of the beta-glucan as rheology modifier

**Figure 5**

## Description

The present invention refers to a process for the fermentation of *Ascomycota,* a beta-glucan produced by the process and the use of the beta-glucan as rheology modifier.

*Ascomycetes* are yeast-like fungi capable of producing a wide variety of different valuable bioproducts such as enzymes, pigments and biopolymers such as beta-glucans. These beta-glucans are of high economic importance as they can be widely used in food, pharmaceutical, agricultural and also chemical applications.

However, fermentation of these fungi has been proven challenging. So far, approaches from multiple disciplines such as microbiology, biochemical engineering, process engineering and genetics have aimed to meet the need of providing a stable and reliable process which achieves beta-glucan yields necessary for industrial scale production. Zhenming et al. (Bioproducts from Aureobasidium pullulans, a biotechnologically important yeast; Appl Microbiol Biotechnol 2009, 82; 793-804) evaluated the effect of various sugar sources and specific enzyme activities on fermentation. In view of the encountered difficulties the authors came to the conclusion that genetic modifications will be a more promising way forward to reach an effective industrial production scale, but this would require a full genome analysis.

The inventors of the present inventions set themselves the task to develop a fermentation method for fungi belonging to the division of *Ascomycota* which leads to sufficiently high yields of the desired fermentation products, especially beta-glucans, while keeping costs at a level suitable for industrial scale production.

This task was solved by a process for the fermentation of Ascomycota comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the division of Ascomycota with an inoculation density selected from the range of from 0.01 $g_{CDW}$/L to 50 $g_{CDW}$/L;

c) Adding at least one carbon source in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.5.

The inventive process guarantees a stable and reliable process which can be implemented in any state of the art fermentation reactor. In addition, the inventive process does not involve genetic engineering or use of genetically modified microorganisms which would increase costs but also limit application of the produced beta-glucans and acceptance by customers. Further, the inventive process guarantees beta glucan yields being more than 3.5 times higher compared to the process under state of the art conditions (see example 1).

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this description and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example", "specific", "specific embodiment"), provided herein is intended merely to better illustrate the invention and does not pose a limitation on the scope of the invention otherwise claimed. No language in the specification should be construed as indicating any non-claimed element essential to the practice of the invention.

Within the present invention the term "Ascomycota" is to be understood to comprise any fungus belonging to the division or phylum of the kingdom Fungi. Ascomycota belong to the subkingdom Dikarya. Ascomycota which are suitable for the inventive process include but are not limited to any fungus falling in the class of Dothideomycetes such as Aureobasidiaceae. The inventive process is particularly suitable for the fermentation of *Aureobasidium pullulans* also referred to as *Aureobasidium oleae, Azymocandida malicola, Candida malicola, Cladosporium pullulans, Dematium pullulans, Exobasidium vitis, Hormonema oleae, Hormonema pullulans, Pullularia fermentans, Pullularia fermentans var. schoenii, Pullularia pullulans, Torula oleae* or *Torula schoenii.* All of which are to be understood as synonyms. Fungi belonging to the division of Ascomycota are commonly referred to as Ascomycetes.

The medium to be provided within step a) of the inventive process is characterized by a dry matter content selected from the range of from 0.1 to 0.3 wt.-%, wherein a dry matter content selected from the range of from 0.15 to 0.25 wt.-% is also within the scope of the present invention. The term "dry matter content" refers to the mass determined after water and other volatile compounds have been removed from the sample using an IR-balance.

At least one of steps c) to g) of the inventive process is carried out at a relative gas content selected from the range of from 0.005 to 0.50, wherein ranges of from 0.0075 to 0.40, from 0.01 to 0.35 and from 0.01 to 0.20 are also within the scope of the present invention.

Within the present invention the relative gas content is defined by the formula:

$$rG = \left(\frac{P}{V_L * \rho_L}\right)^{0,28} * u_G^{\,0,7}$$

(wherein P is the power input [W], $V_L$ is the volume of the medium, the at least one fungus and the at least one carbon source [m³], $\rho_L$ is the density of the medium according to step a), the at least on fungus and the at least one carbon source [kg/m³], and $u_G$ is the superficial gas velocity [m/s] (Liepe (1988): Verfahrenstechnische Berechnungsmethoden Teil 4: Stoffvereinigen in fluiden Phasen - Ausrüstungen und ihre Berechnungen von einem Autorenkollektiv unter Federführung von F. Liepe, Leipzig, VEB Deutscher Verlag für Grundstoffindustrie).

Within the present invention the term "power input" is defined as $P = Ne * \rho_L * n^3 * d^5$ (wherein Ne is the Newton number, $\rho_L$ is the density of the medium according to step a), the at least one fungus and the at least one carbon source [kg/m³], n is the frequency of the stirrer [s⁻¹], d is the diameter of the stirrer [m]). Within the present invention the term superficial gas velocity is defined as

$$u_g = \frac{4 * Q_G}{Dr^2 * \pi},$$

wherein $Q_G$ is the volumetric gas flow [m³/s], to be understood as the volume of air sparged into the medium, the at least on fungus and the at least one carbon source per second, and Dr is the inner diameter of the vessel.

The medium may be provided in any vessel known to a person skilled in the art as suitable for the inventive process, such as a batch or fed-batch reactor, an air lift reactor, a stirred tank reactor or a bubble column reactor. Within a specific embodiment of the present invention, the absolute pressure in the headspace of the vessel is selected from the range of from 1 to 11 bar, wherein ranges of from 1.1 to 10 bar, from 1.2 to 9 bar and from 1.3 to 8 bar are also within the scope of the present invention. Within another specific embodiment of the present invention the vessel is equipped with a light source providing a light intensity of at least 300 Lux. Within a suitable embodiment the light source may be placed above the surface of the fermentation medium during at least 40 % of the time as described in step d). 1 Lux is defined as 1 Lumen per m². Within another exemplary suitable embodiment the light source may be placed next to and outside of the vessel. In this case the vessel may be equipped with a window or may comprise or consist of transparent material. Within another exemplary suitable embodiment the light source may be placed inside the vessel submerged in the fermentation medium.

Within step b) of the inventive process the medium is inoculated with at least one fungus belonging to the division of Ascomycota with an inoculation density selected from the range of from 0.01 $g_{CDW}$/L to 50 $g_{CDW}$/L. The "inoculating" may be carried out by any method known to a person skilled in the art as suitable for the inventive process such as addition of the at least one fungus in a purified form or in form of a pre-culture. Within the inventive process, the concentration of the at least one fungus is selected from a range of from 0.01 $g_{CDW}$/L to 50 $g_{CDW}$/L, wherein ranges of from 0.02 $g_{CDW}$/L to 25 $g_{CDW}$/L, of from 0.03 $g_{CDW}$/L to 20 $g_{CDW}$/L and of from 0.05 $g_{CDW}$/L to 15 $g_{CDW}$/L are also within the scope of the invention. The parameter "L" thereby relates to the volume of the medium according to step a) of the inventive process.

The at least one fungus used in the inventive process is for example only one single type of fungus or a mixture of different fungi. The term "cell dry weight" (CDW) is to be understood as the weight of the total biomass divided by the volume of a fermentation sample after centrifugation of a fermentation sample, removal of the supernatant, washing with a solution of 9 g/L NaCl, again centrifugation, again removal of the supernatant and drying at 90 °C until constant weight is reached.

Within the present invention, the term "pre-culture" is to be understood to comprise any composition of fermentation or culture medium used for pre-cultivating the at least one fungus. The term "pre-culture" is well known to any person skilled in the art of fermenting. Within a specific embodiment, the at least one fungus is pre-cultivated up to a cell dry weight of 2 $g_{CDW}$/L - 30 $g_{CDW}$/L before addition to the medium according to step b) of the inventive process. It is a particular advantage of the inventive process that high yields of beta glucan can be obtained while keeping the volume of pre-culture relatively low, as for example the use of from 0.1 to 0.5 wt.-% (weight pre-culture to weight medium) is sufficient.

Within a special embodiment of the present invention, the nitrogen content of the medium according to step a) is selected from the range of from 0.003 to 0.02 wt.-%, wherein a range of from 0.005 to 0.015 wt.-% and from 0.006 to 0.01 wt.-% is also within the scope of the present invention.

Within a special embodiment of the present invention, step b) of the inventive process is carried out for a time period of from 1 minutes to 8 hours, wherein a time period of from 2 minutes to 7 hours, from 3 minutes to 6 hours and form 5 minutes to 5 hours is also within the scope of the present invention. It is thereby particularly suitable to carry out inoculation by adding the at least one fungus all at once at the beginning of the time period.

Within step c) of the inventive process, at least one carbon source is added in a concentration of from 1 to 20 wt.-%, wherein a concentration of from 2 to 18 wt.-%, from 3 to 15 wt.-% and from 4 to 12 wt.-% is also within the scope of the present invention. The "adding" may be carried out by any method known to a person skilled in the art as suitable for the inventive process. Step c) may be carried out after step b) of the inventive process but may also be carried out before step b) or steps b) and c) may also be carried out concurrently. Within another embodiment of the present invention, steps a) and c) may be carried out concurrently and before step b).

Within step d) of the inventive process the medium and the at least one fungus are mixed for a time period of from 24 to 400 hours, wherein a time period of from 30 to 350 hours, from 35 to 300 hours, from 40 to 250 hours and from 50 to 200 hours are also within the scope of the present invention. The "mixing" may be carried out by any method known to a person skilled in the art as suitable for the inventive process.

A method which has shown to be of specific advantage regarding time and energy consumption is mixing by continuously pumping of the medium and at least one fungus from the bottom to the top of the vessel.

Within a particularly advantageous embodiment, the mixing is carried out without the use of any kind of rotating device such as a stirrer. A system which is advantageous for carrying out the inventive process is for example a bubble column reactor.

Within a specific embodiment, the mixing may be carried out with a specific power input P/V of from 2 to 12.000 W/m$^3$, wherein a specific power input of from 100 to 1.000 W/m$^3$ is also within the scope of the present invention.

Within a stirred mixing system such as a stirred tank reactor, the term "specific power input" is to be understood as

$$\frac{P}{V} = \frac{Ne * \rho_L * n^3 * d^5}{V},$$

wherein P is the power input [W], Ne is the Newton number [-], $\rho$ is the density of the medium according to step a) [kg/m$^3$], n is the stirrer frequency [s$^{-1}$], d is the diameter of the impeller [m] and V is the volume of the medium according to step a), the carbon source and the at least one fungus.

Within a system without any kind of impellor or stirrer such as a bubble column reactor, the specific power input is to be understood as

$$\frac{P}{V} = \rho * g * u_g,$$

wherein g is the gravitational acceleration [m s$^{-2}$], and $u_g$ is the superficial gas velocity as defined above.

Within another specific embodiment, the mixing is carried out until the dynamic viscosity at a shear rate of 20 s$^{-1}$ and at a temperature of 20 °C of the medium and at least one fungus during step d) is within the range of from 40 to 600 mPas, wherein a range of from 50 to 550 mPas, from 70 to 500 mPas, from 90 to 450 mPas and from 100 to 400 mPas is also within the scope of the present invention.

Within a special embodiment of the present invention, the pH is kept in the range of from 4.0 to 6.0 during the whole process, wherein a range of from 4.25 to 5.75, from 4.25 to 5.5 and from 4.25 to 4.75 is also within the scope of the present invention. Within another special embodiment of the present invention, the pH is kept above 4.75, such as within a range of from 4.75 and 5.25 during the complete process by addition of a base, wherein bases can be any bases known to person trained in the skill of fermentation to be suitable for the use in fermentations and not containing nitrogen, such as NaOH or KOH. It is of particular advantage of the inventive process that no acid needs to be used to adjust the pH levels.

Within a special embodiment of the present invention, the temperature is kept in the range of from 22 to 30 °C during the whole process, wherein a range of from 23 to 30 °C, a range from 24 to 29 and from 24 to 28 °C is also within the scope of the present invention.

Within step e) of the inventive process, from 30 to 90 wt.-% of the medium and at least one fungus are removed, wherein ranges of from 40 to 85 wt.-%, from 45 to 80 wt.-%, from 50 to 75 wt.-% and from 55 to 65 wt.-% are also within the scope of the present invention. The "removing" can thereby be carried out by any means and method known to a person skilled in the art as suitable for the inventive process.

Within step f) of the inventive process, from 30 to 90 wt.-% of a medium as defined within step a) of the inventive process and additionally containing a carbon source are added to the remaining medium and at least one fungus, wherein ranges of from 40 to 85 wt.-%, from 45 to 80 wt.-%, from 50 to 75 wt.-% and from 55 to 65 wt.-% are also within the scope of the present invention.

It is thereby understood that the range of from 30 to 90 wt.-% comprises the weight of the medium and the carbon source. The "adding" can thereby be carried out by any means and method known to a person skilled in the art as suitable for the inventive process. It is thereby of importance that the amount of removed medium and fungus and the amount of new medium are equal, wherein a deviation of up to 30%, preferably less than 10 % is tolerable.

Within step g) of the inventive process steps c) to f) are repeated from 2 to 100 times, wherein a repetition of from 2 to 80, from 2 to 70, from 2 to 50 and from 2 to 25 times is also within the scope of the present invention. It is a particular advantage of the inventive process that cells and fermentation products can be harvested up to 100 times without the need of new inoculation. A fermentation process and medium composition as developed by the inventors of the present invention will guarantee high yields of beta glucan. The repetition of steps c) to f) are of particular advantage as the yield of the beta-glucan increases significantly with the first repetition. In addition, cleaning expenditures can be minimized as cleaning of the reactor is only necessary after the completion of step g) of the inventive process contributing to further cost savings, costs for the preparation of precultures can be minimized as preculture preparation is only necessary for every second to 100th iteration of the process. Additionally downtimes (non-productive phases) of the production equipment are minimized, since the production can be kept running without time losses for harvesting, sterilization, cleaning and filling. It is, however, also possible to obtain decent yields of beta glucan by not repeating steps c) to f) but carrying out steps c) to f) just once.

Within the present invention the term "biopolymer" is to be understood as any polymer produced by living organisms. The inventive process and system are particularly suitable for biopolymers which show a temperature-independent viscosity within the range from 20 to 100°C or within the range of from 20 to 80°C. The inventive process is particularly suitable for biopolymers containing monomeric units which are covalently bonded such as polymeric carbohydrate structures, rubber, suberin, melanin and lignin. Within an exemplary embodiment, the at least one biopolymer has a molecular weight of at least 0.8 MegaDalton such as scleroglucan, pullulan or beta-glucan. Within another exemplary embodiment, the at least one biopolymer has a molecular weight distribution with a maximum of from 0.5 to 5.0 Million Dalton (MegaDalton) or from 0.75 to 3.5 Million Dalton (MegaDalton) or from 1.0 to 2.0 Million Dalton (MegaDalton). Within an exemplary embodiment the biopolymer has a shear thinning behavior described by a constant $b$ in which $b$ is the gradient between two pairs of values $x1/y1$ and $x2/y2$ where x is the shear rate $[s^{-1}]$ in the range of 0.1-100 $s^{-1}$ and $y$ is the dynamic viscosity [mPas] at the given shear rate and at a temperature of the biopolymer between 20°C and 80°C and a concentration between 0.05 and 0.5 wt.-%. The constant $b$ can described by the formula $b= -((lg(y1/y2)/(lg(x1/x2))$. Within a suitable but exemplary embodiment b is selected from the range of from 0.65 to 1.05. Within another exemplary embodiment $b$ is selected from the range of from 0.7 to 1.0. Within another exemplary embodiment $b$ is selected from the range of from 0.75 to 0.9. Within the present invention the term "beta-glucan" is to be understood as referring to any β-D-glucose polysaccharide characterized by the formation of a linear backbone with 1-3 β-glycosidic bonds. Within special embodiments of the present invention, the beta-glucans have a molecular weight distribution with a maximum of from 0.5 to 5.0 Million Dalton (MegaDalton) or from 0.75 to 3.5 Million Dalton (MegaDalton) or from 1.0 to 2.0 Million Dalton (MegaDalton).

**Specific embodiments of the present invention**

The following specific embodiments define embodiments which are particularly advantageous for the production of beta-glucan by a fungus belonging to the family of *Aureobasidiaceae.* These embodiments are not meant to limit the scope of the present application in any respect.

Specific embodiment A

Process for the fermentation of *Aureobasidiaceae* comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the species of *Aureobasidium pullulans* with an inoculation density selected from the range of from 0.01 $g_{CDW}$/L to 50 $g_{CDW}$/L;

c) Adding at least one carbon source in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.5.

Specific embodiment B

Process for the fermentation of *Aureobasidiaceae* comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the species of *Aureobasidium pullulans* with an inoculation density selected from the range of from 0.01 $g_{CDW}$/L to 50 $g_{CDW}$/L;

c) Adding sucrose in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.2.

Specific embodiment C

Process for the fermentation of *Aureobasidiaceae* comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the species of *Aureobasidium pullulans* with an inoculation density selected from the range of from 0.01 $g_{CDW}$/L to 50 $g_{CDW}$/L;

c) Adding sucrose in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.2 and at a temperature of from 24 to 30 °C and at a pH of from 4 to 6.

Specific embodiment D

Process for the fermentation of *Aureobasidiaceae* comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the species of *Aureobasidium pullulans* with an inoculation density selected from the range of from 0.01 $g_{CDW}$/L to 50 $g_{CDW}$/L;

c) Adding sucrose in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.2 and at a temperature of from 24 to 30 °C and at a pH of from 4 to 6 and step b) is carried out for a time period of from 1 minute to 8 hours before step c).

**Examples and figures**

In the following, the present invention is described by specific examples and figures. The examples and figures are used for illustrating purposes only and do not limit the scope of the present invention.

List of figures

Fig.1    shows the influence of dry matter and nitrogen content on product formation

Fig.2    shows the influence of temperature on product formation

Fig.3    shows the influence of pH on product formation

Fig.4    shows the influence of the C source on product formation

Fig.5    shows a schematic overview of the repeated batch process

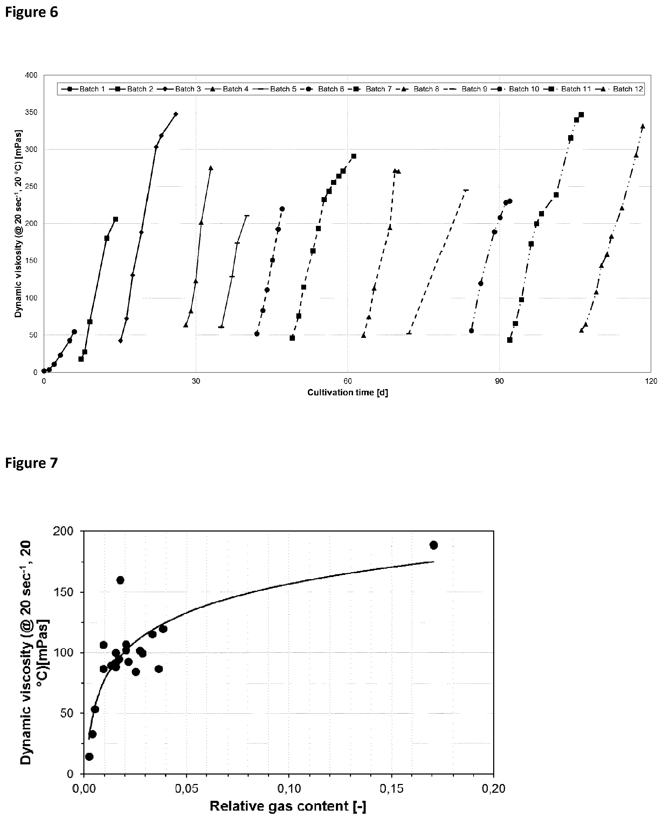

Fig. 6    shows product formation per batch within repeated batch production

Fig. 7    shows the influence of relative gas content on product formation

Fig. 8    shows the influence of a starvation phase on product formation

Example 1:

Influence of dry matter and nitrogen content

100 kg of the following media were prepared and autoclaved at 121 °C for 20 minutes in a Techfors 150-reactor (Infors AG, Bottmingen, Switzerland):

PDB-Medium (Potatoe Dextrose Broth, dry matter content 0.4 wt.-%, nitrogen content 0.008 wt.-%; state of the art): 3 g/kg potatoe extract (Sigma Aldrich, Steinheim, Germany), 1 g/kg Antifoam 204 (Sigma Aldrich, Steinheim, Germany) in tap water (Osin'ska-Jaroszuk et al., Extracellular polysaccharides from Ascomycota and Basidiomycota: production conditions, biochemical characteristics, and biological properties; World J Microbiol Biotechnol. 2015 31:1823-1844),

PM-Medium (Production Medium, dry matter content 0.5 wt.-%, nitrogen content 0.02 wt.-%); state of the art): 2 g/kg (NH4)2SO4 (Sigma Aldrich, Steinheim, Germany), 1 g/kg K2HPO2 (Sigma Aldrich, Steinheim, Germany), 0.5 g/kg KCl (Sigma Aldrich, Steinheim, Germany), 0.5 g/kg MgSO4*7H2O (Sigma Aldrich, Steinheim, Germany), 0.01 g/kg FeSO4*7H2O (Sigma Aldrich, Steinheim, Germany), 0.4 G/kg Yeast extract (Lallemand, Montreal, Canada), 1 g/kg Antifoam 204 in tap water (Youssef et al. Pullulan production by a non-pigmented strain of Aureobasidium pullulans using batch and fed-batch culture; Process Biochemistry 34 (1999) 355-366) and

OM-Medium (invention): 0.4 g/kg NaNO3 (Sigma Aldrich, Steinheim, Germany), 0.4 g/kg K2HPO4, 0.4 g/kg NaCl (Sigma Aldrich, Steinheim, Germany), 0.4 g/kg MgSO4*7H2O (Sigma Aldrich, Steinheim, Germany), 0.2 g/kg yeast extract (Lallemand, Montreal, Canada), 1 g/kg Antifoam 204 (Sigma Aldrich).

Before autoclaving 50 g/kg sucrose (Südzucker AG) were added to each medium. After autoclaving, for the rest of the experiment the temperature was controlled at 26 °C, the pH was adjusted to 4.5 +/- 0.25 using 5 M H2SO4 and 5 M NaOH and controlled to 4.5 +/-0.25 for the remaining fermentation with 5 M NaOH, the medium was stirred and aerated at a relative gas content of 0.016 and a headspace pressure of 0.2 bar.

When constant conditions were reached, each medium was inoculated to a concentration of 0.019 g/kg CDW of *Aureobasidium pullulans.* The organism was cultivated in the respective medium at the conditions mentioned above for 144 h.

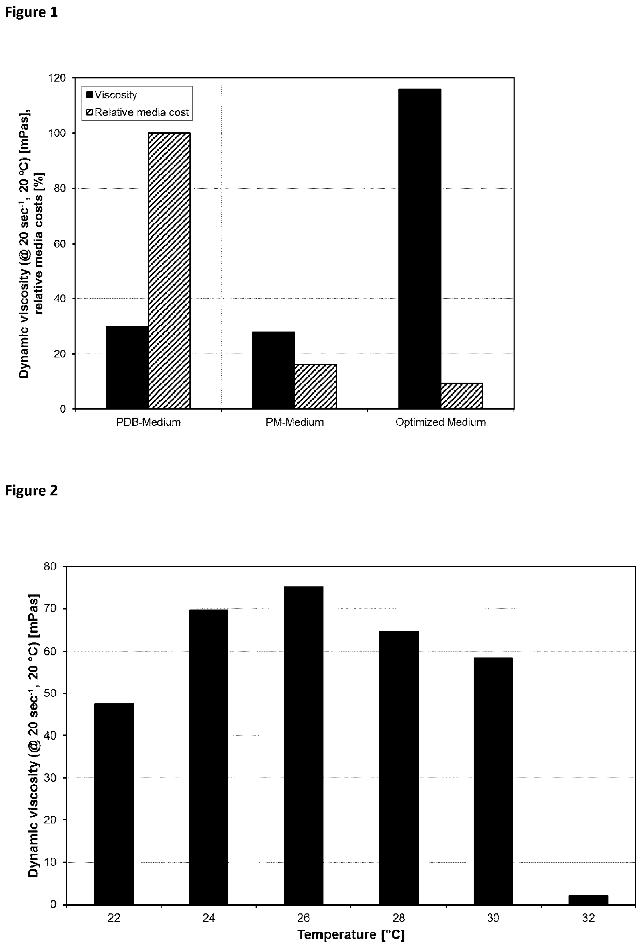

The viscosity as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 s$^{-1}$ and a temperature of 20 °C. The improvement by the self-developed medium is shown in figure 1 by the medium cost (sum of the costs for all medium components) and the dynamic viscosity of the fermentation broth after 144 h of cultivation as a measure of product formation. It is shown that the OM medium with a dry matter content of 0.16 wt.-% and a nitrogen content of 0.0066 wt.-% shows highest viscosity (and therefore beta glucan yield) while keeping costs at a minimum level.

Example 2:

Influence of temperature on beta glucan yield

2 kg of OM medium and 50 g/kg sucrose (Südzucker AG) were autoclaved at 121 °C for 20 minutes in a Labfors 5 BioEtOH-reactor (Infors AG, Bottmingen, Switzerland) equipped with one Rushton turbine and one pitched blade impeller. After autoclaving, for the rest of the experiment the temperature was controlled to the temperatures ranging from 24 to 32 °C (see Fig. 2, the pH was adjusted to 4.5 +/- 0.25 using 5 M H2SO4 and 5 M NaOH and controlled to 4.5 +/-0.25 for the remaining fermentation with 5 M NaOH, the medium was stirred at and aerated at a relative gas content of 0.03.

When constant conditions were reached, the medium was inoculated to a concentration of 0.019 g/kg CDW of *Aureobasidium pullulans.* The organism was cultivated in the respective medium at the conditions mentioned above for 144 h. The viscosity as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 s$^{-1}$ and a temperature of 20 °C.

It is shown in Fig.2 that best results were achieved at temperatures from 24 to 30 °C with an optimum of 26 °C (dynamic viscosity of the fermentation broth after 144 h of cultivation as a measure of beta glucan yield).

Example 3:

Influence of pH on beta glucan yield

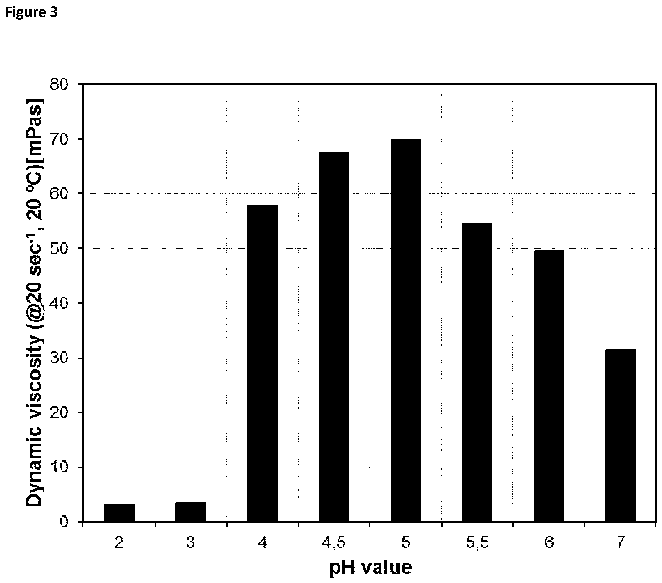

2 kg of OM medium and 50 g/kg sucrose (Südzucker AG) were autoclaved at 121 °C for 20 minutes in a Labfors 5 BioEtOH-reactor (Infors AG, Bottmingen, Switzerland) equipped with one Rushton turbine and one pitched blade impeller. After autoclaving, for the rest of the experiment the temperature was controlled to 26 °C, the pH was adjusted and controlled to the values given in Fig. 3 using 5 M $H_2SO_4$ and 5 M NaOH, the medium was stirred and aerated at a relative gas content of 0.03.

When constant conditions were reached, the medium was inoculated to a concentration of 0.019 g/kg CDW of *Aureobasidium pullulans.* The organism was cultivated in the respective medium at the conditions mentioned above for 144 h. The viscosity as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 $s^{-1}$ and a temperature of 20 °C.

It is shown in Fig. 3 that best results were achieved at a pH from 4 to 6 with an optimum of 5 (dynamic viscosity of the fermentation broth after 144 h of cultivation as a measure of beta glucan yield).

The improvement of the pH set to 5.0 is shown in Fig. 3 by the dynamic viscosity of the fermentation broth after 144 h of cultivation as a measure of product formation.

Example 4

Influence of different C-sources on beta glucan yield

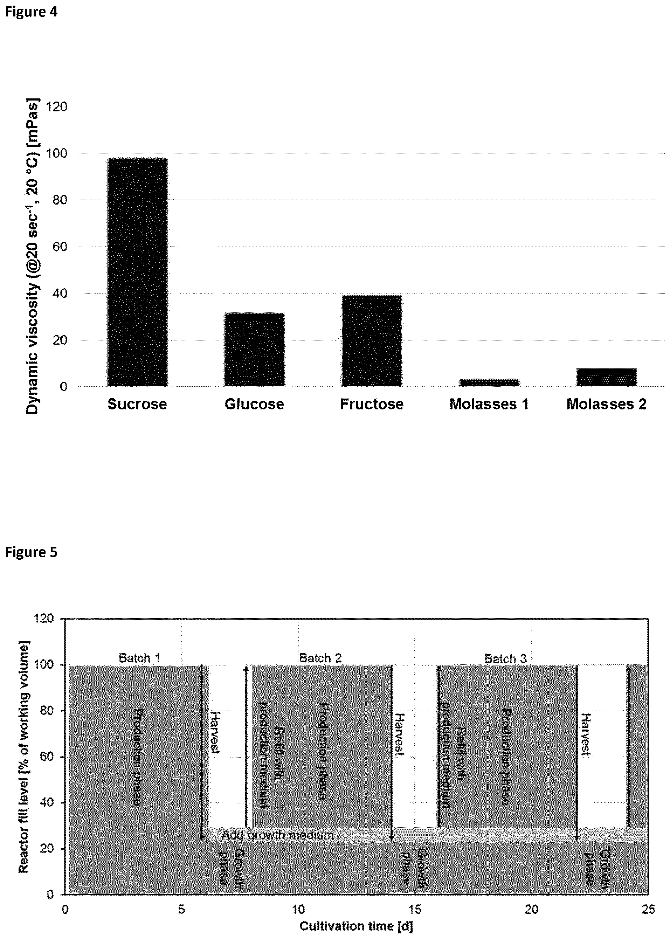

2 kg of OM medium and different C-sources as given in Fig. 4 to equal 50 g total sugar /kg were autoclaved at 121 °C for 20 minutes in a Labfors 5 BioEtOH-reactor (Infors AG, Bottmingen, Switzerland) equipped with one Rushton turbine and one pitched blade impeller. After autoclaving, for the rest of the experiment the temperature was controlled to 26 °C, the pH was adjusted to 4.75 ±0.25 using NaOH and $H_2SO_4$ and controlled to 4.75 ±0.25 using 5 M $H_2SO_4$ and 5 M NaOH, the medium was stirred and aerated at a relative gas content of 0.03.

When constant conditions were reached, the medium was inoculated to a concentration of 0.019 g/kg CDW of *Aureobasidium pullulans.* The organism was cultivated in the respective medium at the conditions mentioned above for 144 h. The viscosity as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 $s^{-1}$ and a temperature of 20 °C.

It is shown in Fig.4 that best results were achieved using sucrose as C source (dynamic viscosity of the fermentation broth after 144 h of cultivation as a measure of beta glucan yield).

Example 5

Repeated batch production

2 kg of OM medium and 50 g/kg sucrose (Südzucker AG) were autoclaved at 121 °C for 20 minutes in a Labfors 5 BioEtOH-reactor (Infors AG, Bottmingen, Switzerland) equipped with one Rushton turbine and one pitched blade impeller. After autoclaving, for the rest of the experiment the temperature was controlled to 26 °C, the pH was adjusted to 4.75 ±0.25 using NaOH and $H_2SO_4$ and controlled to 4.75 ±0.25 using 5 M $H_2SO_4$ and 5 M NaOH, the medium was stirred and aerated at a relative gas content of 0.03.

When constant conditions were reached, the medium was inoculated to a concentration of 0.019 g/kg CDW of *Aureobasidium pullulans.* The organism was cultivated at the conditions mentioned above. When the pH increases above 5, 75 % of the medium were harvested and the fermenter was refilled with fresh OM medium (composition as defined above). In the first nine cycles an additional growth phase was included by adding 5 % of the working volume of a growth medium (30 g/kg sucrose (Südzucker AG) and 20 g/kg yeast extract (Lallemand, Montreal, Canada) for 2 days. In batches 10 to 12 no growth phase was included. This cycle of harvesting and refilling was repeated for several times, as shown in Fig. 5. The viscosity of samples as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 $s^{-1}$ and a temperature of 20 °C.

Example 6

Influence of relative gas content

0.8 L of OM medium and 50 g/kg sucrose (Südzucker AG) were autoclaved at 121 °C for 20 minutes in a DasGip-reactor (DasGip Bioblock stirrer vessel, Eppendorf AG, Hamburg, Germany) equipped with two Rushton turbines, or 2 L of OM medium and 50 g/kg sucrose were autoclaved at 121 °C for 20 minutes in a Labfors 5 BioEtOH-reactor (Infors AG, Bottmingen, Switzerland) equipped with one Rushton turbine and one pitched blade impeller and one pitched blade impeller, or 100 kg of OM medium and 50 g/kg sucrose (Südzucker AG) were autoclaved at 121 °C for 20 minutes in a Techfors 150-reactor (Infors AG, Bottmingen, Switzerland), or 25 m$^3$ of OM medium and 50 g/kg sucrose (Südzucker AG) were sterilized by ultra-high temperature treatment (150 °C, 10 minutes) and transferred into a 30 m$^3$ stirred tank reactor. For the rest of the experiment the temperature was controlled to 26 °C, the pH was adjusted to 4.75 ±0.25 using NaOH and H2SO4 and controlled to 4.75 ±0.25 using 5 M H2SO4 and 5 M NaOH, the medium was stirred at specific power inputs ranging from 100 to 12.000 W/m$^3$ and aerated with 0.5 to 1.5 volumes of air per volume of medium and per minute.

When constant conditions were reached, the medium was inoculated to a concentration of 0.019 g/kg CDW of *Aureobasidium pullulans.* The organism was cultivated at the conditions mentioned above.

Example 7

Influence of starvation phase

2 kg of OM medium (0.4 g/kg NaNO3 (Sigma Aldrich, Steinheim, Germany), 0.4 g/kg K2HPO4, 0.4 g/kg NaCl (Sigma Aldrich, Steinheim, Germany), 0.4 g/kg MgSO4*7H2O (Sigma Aldrich, Steinheim, Germany), 0.2 g/kg yeast extract (Lallemand, Montral, Canada), 1 g/kg Antifoam 204 (Sigma Aldrich)) were autoclaved at 121 °C for 20 minutes in a Labfors 5 BioEtOH-reactor (Infors AG, Bottmingen, Switzerland) equipped with one Rushton turbine and one pitched blade impeller. After autoclaving, for the rest of the experiment the temperature was controlled to 26 °C, the pH was adjusted and controlled to 4.75 using 5 M H2SO4 and 5 M NaOH, the medium was stirred and aerated at a relative gas content of 0.017.

When constant conditions were reached, the medium was inoculated to a concentration of 0.019 g/kg CDW of *Aureobasidium pullulans.* The organism was cultivated in the respective medium at the conditions mentioned above for 144 h. After 0 h, 2 h and 4 h, respectively, a sterile sucrose solution was added to the medium in a single shot to reach a sucrose concentration of 50 g/kg. The viscosity as a measure for the product formation was measured with a rotational rheometer and coaxial cylinder according to DIN 53019 using a Malvern Kinexus Lab+-rheometer (Malvern Panalytical Ltd., Almelo, Netherlands) at a shear rate of 20 s$^{-1}$ and a temperature of 20 °C.

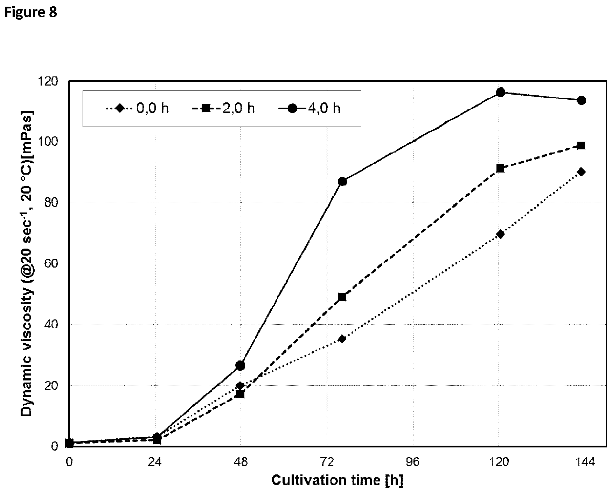

It is shown in Fig. 8 that best results were achieved implementing a starvation phase of 4 hours (dynamic viscosity of the fermentation broth after 144 h of cultivation as a measure of beta glucan yield).

**Claims**

**1.** Process for the fermentation of Ascomycota comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the division of Ascomycota with an inoculation density selected from the range of from 0.01 g$_{CDW}$/L to 50 g$_{CDW}$/L;

c) Adding at least one carbon source in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times;

wherein at least one of steps c) to g) is carried out with a relative gas content selected from the range of from 0.005 to 0.5.

**2.** Process according to claim 1, wherein the cell dry weight CDW of the medium in step b) is selected from the range of from 0.01 to 50 g$_{CDW}$/L.

**3.** Process according to any of the foregoing claims, wherein the nitrogen content of the medium according to step a) is selected from the range of from 0.003 to 0.02 wt.-%.

**4.** Process according to any of the foregoing claims, wherein the pH is kept in the range of from 4.0 to 6.0 during the whole process.

**5.** Process according to any of the foregoing claims, wherein the temperature T is kept in the range of from 22 to 30 °C during the whole process.

**6.** Process according to any of the foregoing claims, wherein the dynamic viscosity of the medium during step d) is selected from the range of from 40 to 600 mPas.

**7.** Process according to any of the foregoing claims, wherein step b) is carried out for a time period of from 1 minute to 8 hours.

**8.** Beta-glucan produced by a process according to any of claims 1 to 7.

**Figure 1**

**Figure 2**

Figure 3

EP 3 550 027 A1

**Figure 4**

**Figure 5**

12

Figure 6

Figure 7

**Figure 8**

Europäisches
Patentamt

European
Patent Office

Office européen
des brevets

**EUROPEAN SEARCH REPORT**

Application Number

EP 18 16 5787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEKAR ET AL: "Strain selection and optimization of mixed culture conditions for Lactobacillus pentosus K1-23 with antibacterial activity and Aureobasidium pullulans NRRL 58012 producing immune-enhancing beta-glucans", JOURNAL OF MICROBIOLOGY AND BIOTECHNOLOGY, vol. 28, 16 March 2018 (2018-03-16), pages 697-706, XP002785030, * See page 701 (Table 3); early online publication * | 1-8 | INV. C12P19/04 |
| A | KIM ET AL: "Optimization of beta-1,3/1,6-glucan production of Aureobasidium pullulans KSY-0516 strain", JOURNAL OF THE KOREAN SOCIETY OF FOOD SCIENCE AND NUTRITION, vol. 46, 2017, pages 1568-1573, XP002785113, * See page 1568 (Abstract) * | 1-8 | |
| A | PRASONGSUK ET AL: "The current status of Aureobasidium pullulans in biotechnology", FOLIA MICROBIOLOGY, vol. 63, 27 October 2017 (2017-10-27), pages 129-140, XP036424203, * See pages 136-137 (Aubasidan-like beta-glucan); early online publication * | 1-8 | TECHNICAL FIELDS SEARCHED (IPC)<br><br>C12P |

-/--

The present search report has been drawn up for all claims

| Place of search | Date of completion of the search | Examiner |
|---|---|---|
| Berlin | 26 September 2018 | Korsner, Sven-Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
.................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 16 5787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MORIYA ET AL: "Improved beta-glucan yield using an Aureobasidium pullulans M-2 mutant strain in a 200-L pilot scale fermentor targeting industrial mass production", BIOTECHNOLOGY AND BIOPROCESS ENGINEERING, vol. 18, 2013, pages 1083-1089, XP002785031, * See pages 1087-1089 (sections 3.5-3.6) * | 1-8 | |
| A | CROGNALE ET AL: "Production of beta-glucan and related glucan-hydrolases by Botryosphaeria rhodina", JOURNAL OF APPLIED MICROBIOLOGY, vol. 102, 2007, pages 860-871, XP002785114, * See pages 869-870 (Discussion) * | 1-8 | |
| L | US 9 938 550 B1 (HUANG ET AL) 10 April 2018 (2018-04-10) * See columns 1-3 (Summary) and Figures 2-7 * | | TECHNICAL FIELDS SEARCHED (IPC) |

The present search report has been drawn up for all claims

| Place of search | Date of completion of the search | Examiner |
|---|---|---|
| Berlin | 26 September 2018 | Korsner, Sven-Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 18 16 5787

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2018

| Patent document cited in search report | Publication date | Patent family member(s) | Publication date |
|---|---|---|---|
| US 9938550 B1 | 10-04-2018 | CN 108118003 A<br>JP 2018088909 A<br>TW 201821611 A<br>US 9938550 B1 | 05-06-2018<br>14-06-2018<br>16-06-2018<br>10-04-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHENMING et al.** Bioproducts from Aureobasidium pullulans, a biotechnologically important yeast. *Appl Microbiol Biotechnol,* 2009, vol. 82, 793-804
- **OSIN'SKA-JAROSZUK et al.** Extracellular polysaccharides from Ascomycota and Basidiomycota: production conditions, biochemical characteristics, and biological properties. *World J Microbiol Biotechnol.,* 2015, vol. 31, 1823-1844

- **YOUSSEF et al.** Pullulan production by a non-pigmented strain of Aureobasidium pullulans using batch and fed-batch culture. *Process Biochemistry,* 1999, vol. 34, 355-366